# EUROPEAN PATENT APPLICATION

(11) **EP 3 309 156 A1**
(43) Date of publication of application: **18.04.2018**
(21) Application number: 16193814.7
(22) Date of filing: 13.10.2016
(51) Int. Cl.: C07D 405/12, A61K 31/4525, A61P 33/06

(54) **NEW ANTI-MALARIAL AGENTS**

(71) Applicant: MMV Medicines for Malaria Venture, 1215 Geneva (CH)
(72) Inventor: VENNERSTROM, Jonathan Lee, Omaha, NE Nebraska 68131 (US); CHARMAN, Susan Ann, SOUTH MELBOURNE, Victoria 3205 (AU)
(74) Representative: reuteler & cie SA

(57) **Abstract**

The present invention is related to new trioxolane derivatives of formula (I) in the manufacture of a medicament for preventing or treating malaria. Specifically, the present invention is related to trioxolane derivatives useful for the preparation of a pharmaceutical formulation for the inhibition of malaria parasite proliferation.

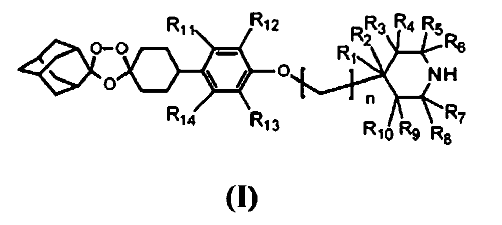

## Description

### Field of the Invention

The present invention relates to novel anti-malarial agents. Specifically, the present invention is related to agents useful for the preparation of a pharmaceutical formulation for preventing or treating malaria and methods of their use and manufacture.

### Background of the Invention

Malaria is caused by protozoan parasites of the genus *Plasmodium* that infect and destroy red blood cells, leading to fever, severe anemia, cerebral malaria and, if untreated, death. *Plasmodium falciparum* is the dominant species in sub-Saharan Africa, and is responsible for almost 1 million deaths each year. The disease burden is heaviest in African children under 5 years of age and in pregnant women. *Plasmodium vivax* causes 25-40% of the global malaria burden, particularly in South and Southeast Asia, and Central and South America. The other two main species that are known to infect humans are *Plasmodium ovale, Plasmodium malariae and Plasmodium knowlesi.*

Malaria is a disease that is prevalent in many developing countries. Approximately 40% of the world's population lives in countries where the disease is endemic; approximately 247 million people suffer from the disease every year.

Antimalarial drugs are key tools for the control and elimination of malaria. Recent decreases in the global malaria burden are likely due, in part, to the deployment of artemisinin-based combination therapies. However, the increase in artemisinin-resistant parasites in southeast Asia, their potential spread to other malaria-endemic regions and changes in sensitivities to artemisinin partner drugs have raised concerns since it could lead to loss of treatment efficacy and in the worst case lead to an epidemic of drug resistant malaria, unless alternative compounds are identified (Cui et al., 2015, Am. J. Trop. Med. Hyg., 93(Suppl 3), 57-68). Various chemical agents have been developed for the treatment and prevention of malaria over the past 20 years (Wells et al., 2015, Nature Reviews Drug Discovery 14, 424-442)*.* Among those, spiro and dispiro 1,2,4-trioxolane compounds have been investigated as antimalarials (WO 09/058859 and WO 09/091433) and are still under development, having shown a greatly higher safety margin as compared to artemisinin (Nigussie et al., 2015, Malaria Contr. Elimination, SI: SI-007. doi:10.41721/1000S1-007*,-* Charman et al., 2011, PNAS, 108 (11), 4400-4405*).*

Various medications are presently used for the treatment of malaria. However, many of these medications are costly and some exhibit significant toxicity and undesirable side effects in humans. Drugs used for treating malaria include artemisinin and its derivatives such as artemether, artesunate, dihydroartemisinin, and also chloroquine, hydroxychloroquine, quinine, mefloquine, amodiaquine, atovaquone/proguanil, doxycycline, clindamycin, halofantrine, lumefantrine, pyronaridine, pyrimethamine-sulfadoxine, piperaquine and primaquine.

However, current treatments must be taken over 3 days and studies suggest that patient adherence is low which can lead to incomplete cure and contribute to the spread of resistant pathogen strains.

Therefore, the widespread emergence of drug resistance of malaria parasites in endemic countries has compromised many of the current chemotherapies and there is a continued need for new chemotherapeutic approaches. Accordingly, this invention provides novel potent anti-malarial agents and methodology of treating malaria using novel potent anti-malarial agents.

### Summary of the Invention

The present invention is directed towards novel dispiro 1,2,4-trioxolane derivatives which are useful in the treatment and/or prophylaxis of malaria, pharmaceutical formulation, use and manufacture thereof.

A first aspect of the invention provides a dispiro 1,2,4-trioxolane derivative according to the invention or a pharmaceutically acceptable salt thereof or a pharmaceutically active derivative thereof.

Another aspect of the invention relates to a dispiro 1,2,4-trioxolane derivative or a pharmaceutically acceptable salt thereof or a pharmaceutically active derivative thereof according to the invention for use as a medicament.

Another aspect of the invention relates to a dispiro 1,2,4-trioxolane derivative according to the invention or a pharmaceutically acceptable salt thereof or a pharmaceutically active derivative thereof for use in the prevention and/or treatment of malaria.

Another aspect of the invention relates to a dispiro 1,2,4-trioxolane derivative according to the invention or a pharmaceutically acceptable salt thereof or a pharmaceutically active derivative thereof for use in the prevention and/or treatment of schistosomiasis or a cancer.

Another aspect of the invention relates to the use of a dispiro 1,2,4-trioxolane derivative according to the invention or a pharmaceutically acceptable salt thereof or a pharmaceutically active derivative thereof for the preparation of a pharmaceutical composition for the prevention and/or treatment of malaria.

Another aspect of the invention relates to the use of a dispiro 1,2,4-trioxolane derivative according to the invention or a pharmaceutically acceptable salt thereof or a pharmaceutically active derivative thereof for the preparation of a pharmaceutical composition for the prevention and/or treatment of schistosomiasis or a cancer.

Another aspect of the invention resides in a pharmaceutical formulation comprising at least one dispiro 1,2,4-trioxolane derivative according to the invention or a pharmaceutically acceptable salt thereof or a pharmaceutically active derivative thereof and a pharmaceutically acceptable carrier, diluent or excipient thereof.

Another aspect of the invention resides in a method for preventing and/or treating malaria in a subject. The method comprises administering a dispiro 1,2,4-trioxolane derivative according to the invention or a pharmaceutically acceptable salt thereof or a pharmaceutically active derivative thereof in a subject in need thereof.

Another aspect of the invention resides in a method for preventing and/or treating schistosomiasis or a cancer in a subject. The method comprises administering a dispiro 1,2,4-trioxolane derivative according to the invention or a pharmaceutically acceptable salt thereof or a pharmaceutically active derivative thereof in a subject in need thereof

Another aspect of the invention provides a process for the preparation of a dispiro 1,2,4-trioxolane derivative according to the invention or a pharmaceutically acceptable salt thereof or a pharmaceutically active derivative thereof according to the invention and intermediates thereof.

Another aspect of the invention provides a process for the preparation of a compound of Formula (I) comprising a step of reacting an intermediate of Formula (v) or an intermediate of Formula (iii).

Another aspect of the invention provides an intermediate of Formula (v) according to the invention.

Other features and advantages of the invention will be apparent from the following detailed description.

### Detailed Description of the invention

The following paragraphs provide definitions of the various chemical moieties that make up the compounds according to the invention and are intended to apply uniformly through-out the specification and claims, unless an otherwise expressly set out definition provides a broader definition.

The term "C₁-C₆ alkyl" when used alone or in combination with other terms, comprises a straight chain or branched C₁-C₆ alkyl which refers to monovalent alkyl groups having 1 to 6 carbon atoms. This term is exemplified by groups such as methyl, ethyl, n-propyl, i-propyl, n-butyl, s-butyl, i-butyl, t-butyl, n-pentyl, 1-ethylpropyl, 2-methylbutyl, 3-methylbutyl, 2,2-dimethylpropyl, n-hexyl, 2-methylpentyl, 3-methylpentyl, 4-methylpentyl, and the like.

The term "C₂-C₆ alkenyl" when used alone or in combination with other terms, comprises a straight chain or branched C₂-C₆ alkenyl. Particularly, it refers to groups having 2 to 6 carbon atoms and having at least 1 or 2 sites of alkenyl unsaturation. It may have any available number of double bonds in any available positions, and the configuration of the double bond may be the (E) or (Z) configuration. This term is exemplified by groups such as vinyl, allyl, isopropenyl, 1-propenyl, 2-methyl-1-propenyl, 1-butenyl, 2-butenyl, 3-butenyl, 2-ethyl-1-butenyl, 3-methyl-2-butenyl, 1-pentenyl, 2-pentenyl, 3-pentenyl, 4-pentenyl, 4-methyl-3-pentenyl, 1-hexenyl, 2-hexenyl, 3-hexenyl, 4-hexenyl, 5-hexenyl, and the like. Among others, are vinyl or ethenyl (-CH=CH₂), n-2-propenyl (allyl, -CH₂CH=CH₂), isopropenyl, 1-propenyl, 2-methyl-1-propenyl, 1-butenyl, 2-butenyl, and 3-methyl-2-butenyl and the like.

The term " C₂-C₆ alkynyl" when used alone or in combination with other terms, comprises a straight chain or branched C₂-C₆ alkynyl. It may have any available number of triple bonds in any available positions. This term is exemplified by groups such as alkynyl groups that may have a carbon number of 2-6, and optionally a double bond, such as ethynyl (-C≡CH), 1-propynyl, 2-propynyl (propargyl: -CH₂C≡CH), 2-butynyl, 2-pentene-4-ynyl, and the like. The term "heteroalkyl" refers to C₁-C₁₂ -alkyl, preferably C₁-C₆ -alkyl, wherein at least one carbon has been replaced by a heteroatom selected from O, N or S, including 2-methoxy ethyl and the like.

The term "aryl" refers to an unsaturated aromatic carbocyclic group of from 6 to 14 carbon atoms having a single ring (*e.g.* phenyl) or multiple condensed rings (*e.g.,* indenyl, naphthyl). Aryl include phenyl, naphthyl, anthryl, phenanthrenyl and the like.

The term "C₁-C₆ alkyl aryl" refers to aryl groups having a C₁-C₆ alkyl substituent, including methyl phenyl, ethyl phenyl and the like.

The term "aryl C₁-C₆ alkyl" refers to C₁-C₆ alkyl groups having an aryl substituent, including 3-phenylpropanyl, benzyl and the like.

The term "heteroaryl" refers to a monocyclic heteroaromatic, or a bicyclic or a tricyclic fused-ring heteroaromatic group. Particular examples of heteroaromatic groups include optionally substituted pyridyl, pyrrolyl, pyrimidinyl, furyl, thienyl, imidazolyl, oxazolyl, isoxazolyl, thiazolyl, isothiazolyl, pyrazolyl, 1,2,3-triazolyl, 1,2,4-triazolyl, 1,2,3-oxadiazolyl, 1,2,4-oxadiazolyl, 1,2,5-oxadiazolyl, 1,3,4-oxadiazolyl,1,3,4-triazinyl, 1,2,3-triazinyl, benzofuryl, [2,3-dihydro]benzofuryl, isobenzofuryl, benzothienyl, benzotriazolyl, isobenzothienyl, indolyl, isoindolyl, isoquinolinyl, 3H-indolyl, benzimidazolyl, imidazo[1,2-a]pyridyl, benzothiazolyl, benzoxazolyl, quinolizinyl, quinazolinyl, pthalazinyl, quinoxalinyl, cinnolinyl, napthyridinyl, pyrido[3,4-b]pyridyl, pyrido[3,2-b]pyridyl, pyrido[4,3-b]pyridyl, quinolyl, isoquinolyl, tetrazolyl, 5,6,7,8-tetrahydroquinolyl, 5,6,7,8-tetrahydroisoquinolyl, purinyl, pteridinyl, carbazolyl, xanthenyl or benzoquinolyl.

The term "C₁-C₆ alkyl heteroaryl" refers to heteroaryl groups having a C₁-C₆ alkyl substituent, including methyl furyl and the like.

The term "heteroaryl C₁-C₆ alkyl" refers to C₁-C₆ alkyl groups having a heteroaryl substituent, including furyl methyl and the like.

The term "C₂-C₆ alkenyl aryl" refers to an aryl groups having a C₂-C₆ alkenyl substituent, including vinyl phenyl and the like.

The term "aryl C₂-C₆ alkenyl" refers to a C₂-C₆ alkenyl groups having an aryl substituent, including phenyl vinyl and the like.

The term "C₂-C₆ alkenyl heteroaryl" refers to heteroaryl groups having a C₂-C₆ alkenyl substituent, including vinyl pyridinyl and the like.

The term "heteroaryl C₂-C₆ alkenyl" refers to C₂-C₆ alkenyl groups having a heteroaryl substituent, including pyridinyl vinyl and the like.

The term "C₃-C₈-cycloalkyl" refers to a saturated carbocyclic group of from 3 to 8 carbon atoms having a single ring (*e.g.* cyclohexyl) or multiple condensed rings (*e.g.* norbornyl). C₃-C₈-cycloalkyl includes cyclopentyl, cyclohexyl, norbornyl and the like.

The term "heterocycloalkyl" refers to a C₃-C₈-cycloalkyl group according to the definition above, in which up to 3 carbon atoms are replaced by heteroatoms chosen from the group consisting of O, S, NR, R being defined as hydrogen or methyl. Heterocycloalkyl include pyrrolidinyl, piperidinyl, piperazinyl, morpholinyl, tetrahydrofuranyl and the like.

The term "C₁-C₆ alkyl C₃-C₈-cycloalkyl" refers to C₃-C₈-cycloalkyl groups having a C₁-C₆ alkyl substituent, including methyl cyclopentyl and the like.

The term "C₃-C₈-cycloalkyl C₁-C₆ alkyl" refers to C₁-C₆ alkyl groups having a C₃-C₈-cycloalkyl substituent, including 3-cyclopentyl propyl and the like.

The term "C₁-C₆ alkyl heterocycloalkyl" refers to heterocycloalkyl groups having a C₁-C₆ alkyl substituent, including 4-methylpiperidinyl and the like.

The term "heterocycloalkyl C₁-C₆ alkyl" refers to C₁-C₆ alkyl groups having a heterocycloalkyl substituent, including (1-methylpiperidin-4-yl) methyl and the like.

The term "carboxy" refers to the group -C(O)OH.

The term "carboxy C₁-C₆ alkyl" refers to C₁-C₆ alkyl groups having a carboxy substituent, including 2-carboxyethyl and the like.

The term "acyl" refers to the group -C(O)R where R includes H, " C₁-C₆ alkyl," "aryl," "heteroaryl," "C₃-C₈-cycloalkyl," "heterocycloalkyl," "aryl C₁-C₆ alkyl," "heteroaryl C₁-C₆ alkyl," "C₃-C₈-cycloalkyl C₁-C₆ alkyl" or "heterocycloalkyl C₁-C₆ alkyl", including acetyl and the like.

The term "acyl C₁-C₆ alkyl" to C₁-C₆ alkyl groups having an acyl substituent, including 2-acetylethyl and the like.

The term "acyl aryl" refers to aryl groups having an acyl substituent, including 2-acetylphenyl and the like.

The term "acyloxy" refers to the group -OC(O)R where R includes H, "C₁-C₆ alkyl", "C₂-C₆ alkenyl," "C₂-C₆ alkynyl," "C₃-C₈-cycloalkyl," "heterocycloalkyl," "aryl," "heteroaryl," "aryl C₁-C₆ alkyl", "heteroaryl C₁-C₆ alkyl," "aryl C₂-C₆ alkenyl," "heteroaryl C₂-C₆ alkenyl," "aryl C₂-C₆ alkynyl," "heteroaryl C₂-C₆ alkynyl," "C₃-C₈-cycloalkyl C₁-C₆ alkyl," or "heterocycloalkyl C₁-C₆ alkyl", including acetyloxy and the like.

The term "acyloxy C₁-C₆ alkyl" refers to alkyl groups having an acyloxy substituent, including 2-(ethylcarbonyloxy)ethyl and the like.

The term "alkoxy" refers to the group -O-R where R includes optionally substituted "C₁-C₆ alkyl", optionally substituted "aryl", optionally substituted "heteroaryl", optionally substituted "aryl C₁-C₆ alkyl" or optionally substituted "heteroaryl C₁-C₆ alkyl".

The term "alkoxy C₁-C₆ alkyl" refers to C₁-C₆ alkyl groups having an alkoxy substituent, including methoxyethyl and the like.

The term "alkoxycarbonyl" refers to the group -C(O)OR where R includes "C₁-C₆ alkyl", "aryl", "heteroaryl" , "aryl C₁-C₆ alkyl", "heteroaryl C₁-C₆ alkyl" or "heteroalkyl".

The term "alkoxycarbonyl C₁-C₆ alkyl" refers to C₁-C₆ alkyl groups having an alkoxycarbonyl substituent, including 2-(benzyloxycarbonyl)ethyl and the like.

The term "aminocarbonyl" refers to the group -C(O)NRR' where R and R' are independently H, C₁-C₆ alkyl, aryl, heteroaryl, "aryl C₁-C₆ alkyl" or "heteroaryl C₁-C₆ alkyl," including N-phenyl carbonyl and the like.

The term "aminocarbonyl C₁-C₆ alkyl" refers to C₁-C₆ alkyl groups having an aminocarbonyl substituent, including 2-(dimethylaminocarbonyl)ethyl, N-ethyl acetamidyl, N,N-Diethyl-acetamidyl and the like.

The term "acylamino" refers to the group -NRC(O)R' where R and R' are independently H, "C₁-C₆ alkyl," "C₂-C₆ alkenyl," "C₂-C₆ alkynyl," "C₃-C₈-cycloalkyl," "heterocycloalkyl," "aryl," "heteroaryl," "aryl C₁-C₆ alkyl", "heteroaryl C₁-C₆ alkyl," "aryl C₂-C₆ alkenyl," "heteroaryl C₂-C₆ alkenyl," "aryl C₂-C₆ alkynyl," "heteroaryl C₂-C₆ alkynyl," "C₃-C₈-cycloalkyl C₁-C₆ alkyl," or "heterocycloalkyl C₁-C₆ alkyl", including acetylamino and the like.

The term "acylamino C₁-C₆ alkyl" refers to C₁-C₆ alkyl groups having an acylamino substituent, including 2-(propionylamino)ethyl and the like.

The term "ureido" refers to the group -NRC(O)NR'R" where R, R and R" are independently H, "C₁-C₆ alkyl," "C₂-C₆ alkenyl," "C₂-C₆ alkynyl," "C₃-C₈-cycloalkyl," "heterocycloalkyl," "aryl," "heteroaryl," "aryl C₁-C₆ alkyl", "heteroaryl C₁-C₆ alkyl," "aryl C₂-C₆ alkenyl," "heteroaryl C₂-C₆ alkenyl," "aryl C₂-C₆ alkynyl," "heteroaryl C₂-C₆ alkynyl," "C₃-C₈-cycloalkyl C₂-C₆ alkyl," or "heterocycloalkyl C₁-C₆ alkyl," and where R' and R," together with the nitrogen atom to which they are attached, can optionally form a 3-8-membered heterocycloalkyl ring.

The term "ureido C₁-C₆ alkyl" refers to C₁-C₆ -alkyl groups having an ureido substituent, including 2-(*N*'-methylureido)ethyl and the like.

The term "carbamate" refers to the group -NRC(O)OR' where R and R' are independently "C₁-C₆ alkyl," "C₂-C₆ alkenyl," "C₂-C₆ alkynyl," "C₃-C₈-cycloalkyl," "heterocycloalkyl," "aryl," "heteroaryl," "C₁-C₆ alkyl aryl" , "heteroaryl C₁-C₆ alkyl," "aryl C₂-C₆ alkenyl," "heteroaryl C₂-C₆ alkenyl," "aryl C₂-C₆ alkynyl," "heteroaryl C₂-C₆ alkynyl," "C₃-C₈-cycloalkyl C₁-C₆ alkyl," or "heterocycloalkyl C₁-C₆ alkyl" and optionally R can also be hydrogen.

The term "amino" refers to the group -NRR' where R and R' are independently H , "C₁-C₆ alkyl", "aryl", "heteroaryl", "C₁-C₆ alkyl aryl", "C₁-C₆ alkyl heteroaryl," "C₃-C₈-cycloalkyl," or "heterocycloalkyl," and where R and R', together with the nitrogen atom to which they are attached, can optionally form a 3-8-membered heterocycloalkyl ring.

The term "amino C₁-C₆ alkyl" refers to alkyl groups having an amino substituent, including 2-(1-pyrrolidinyl)ethyl and the like.

The term "ammonium" refers to a positively charged group -N⁺RR'R" where R, R' and R" are independently "C₁-C₆ alkyl", "C₁-C₆ alkyl aryl", "C₁-C₆ alkyl heteroaryl," "C₃-C₈-cycloalkyl," or "heterocycloalkyl," and where R and R', together with the nitrogen atom to which they are attached, can optionally form a 3-8-membered heterocycloalkyl ring.

The term "ammonium C₁-C₆ alkyl" refers to alkyl groups having an ammonium substituent, including 1-ethylpyrrolidinium and the like.

The term "halogen" refers to fluoro, chloro, bromo and iodo atoms.

The term "sulfonyloxy" refers to a group -OSO₂-R wherein R is selected from "C₁-C₆ alkyl," "C₁-C₆ alkyl" substituted with halogens, *e.g*., an -OSO₂-CF₃ group, "C₂-C₆ alkenyl," "C₂-C₆ C₂-C₆ alkynyl," "C₃-C₈-cycloalkyl," "heterocycloalkyl," "aryl," "heteroaryl," "aryl C₁-C₆ alkyl", "heteroaryl C₁-C₆ alkyl," "aryl C₂-C₆ alkenyl," "heteroaryl C₂-C₆ alkenyl," "aryl C₂-C₆ alkynyl," "heteroaryl C₂-C₆ alkynyl," "C₃-C₈-cycloalkyl C₁-C₆ alkyl," or "heterocycloalkyl C₁-C₆ alkyl".

The term "sulfamate" refers to a group -OSO₂-NRR' wherein R and R' are independently selected from H, "C₁-C₆ alkyl," "C₂-C₆ alkenyl," "C₂-C₆ alkynyl," "C₃-C₈-cycloalkyl," "heterocycloalkyl," "aryl," "heteroaryl," "aryl C₁-C₆ alkyl", "heteroaryl C₁-C₆ alkyl," "aryl C₂-C₆ alkenyl," "heteroaryl C₂-C₆ alkenyl," "aryl C₂-C₆ alkynyl," "heteroaryl C₂-C₆ alkynyl," "C₃-C₈-cycloalkyl C₁-C₆ alkyl," or "heterocycloalkyl C₁-C₆ alkyl" and the like.

The term "sulfonyloxy C₁-C₆ alkyl" refers to alkyl groups having a sulfonyloxy substituent, including 2-(methylsulfonyloxy)ethyl and the like.

The term "sulfonyl" refers to group "-SO₂-R" wherein R is selected from "aryl," "heteroaryl," "C₁-C₆ alkyl," "C₁-C₆ alkyl" substituted with halogens, e.g., an -SO₂-CF₃ group, "C₂-C₆ alkenyl," "C₂-C₆ alkynyl," "C₃-C₈-cycloalkyl," "heterocycloalkyl," "aryl," "heteroaryl," "aryl C₁-C₆ alkyl", "heteroaryl C₁-C₆ alkyl," "aryl C₂-C₆ alkenyl," "heteroaryl C₂-C₆ alkenyl," "aryl C₂-C₆ alkynyl," "heteroaryl C₂-C₆ alkynyl," "C₃-C₈-cycloalkyl C₁-C₆ alkyl," or "heterocycloalkyl C₁-C₆ alkyl".

The term "sulfonyl C₁-C₆ alkyl" refers to alkyl groups having a sulfonyl substituent, including 2-(methylsulfonyl)ethyl and the like.

The term "sulfinyl" refers to a group "-S(O)-R" wherein R is selected from "C₁-C₆ alkyl," "C₁-C₆ alkyl" substituted with halogens, *e.g.,* a -SO-CF₃ group, "C₂-C₆ alkenyl," "C₂-C₆ alkynyl," "C₃-C₈-cycloalkyl," "heterocycloalkyl," "aryl," "heteroaryl," "aryl C₁-C₆ alkyl", "heteroaryl C₁-C₆ alkyl," "aryl C₂-C₆ alkenyl," "heteroaryl C₂-C₆ alkenyl," "aryl C₂-C₆ alkynyl," "heteroaryl C₂-C₆ alkynyl," "C₃-C₈-cycloalkyl C₁-C₆ alkyl," or "heterocycloalkyl C₁-C₆ alkyl".

The term "sulfinyl C₁-C₆ alkyl" refers to alkyl groups having a sulfinyl substituent, including 2-(methylsulfinyl)ethyl and the like.

The term "sulfanyl" refers to groups -S-R where R includes H, halogens, e.g. a -SF₅ group, optionally substituted "C₁-C₆ alkyl," in particular "C₁-C₆ alkyl" substituted with halogens, *e.g.,* a -S-CF₃ group, "C₂-C₆ alkenyl," "C₂-C₆ alkynyl," "C₃-C₈-cycloalkyl," "heterocycloalkyl," "aryl," "heteroaryl," "aryl C₁-C₆ alkyl", "heteroaryl C₁-C₆ alkyl," "aryl C₂-C₆ alkenyl," "heteroaryl C₂-C₆ alkenyl," "aryl C₂-C₆ alkynyl," "alkynylheteroaryl," "C₃-C₈-cycloalkyl C₁-C₆ alkyl," or "heterocycloalkyl C₁-C₆ alkyl".

The term "sulfanyl C₁-C₆ alkyl" refers to C₁-C₅-alkyl groups having a sulfanyl substituent, including 2-(ethylsulfanyl)ethyl and the like.

The term "sulfonylamino" refers to a group -NRSO₂-R' where R and R' are independently "C₁-C₆ alkyl," "C₂-C₆ alkenyl," "C₂-C₆ alkynyl," "C₃-C₈-cycloalkyl," "heterocycloalkyl," "aryl," "heteroaryl," "aryl C₁-C₆ alkyl", "heteroaryl C₁-C₆ alkyl," "aryl C₂-C₆ alkenyl," "heteroaryl C₂-C₆ alkenyl," "aryl C₂-C₆ alkynyl," "heteroaryl C₂-C₆ alkynyl," "C₃-C₈-cycloalkyl C₁-C₆ alkyl," or "heterocycloalkyl C₁-C₆ alkyl".

The term "sulfonylamino C₁-C₆ alkyl" refers to alkyl groups having a sulfonylamino substituent, including 2-(ethylsulfonylamino)ethyl and the like.

The term "aminosulfonyl" refers to a group -SO₂-NRR' where R and R' are independently H, "C₁-C₆ alkyl," "C₂-C₆ alkenyl," "C₂-C₆ alkynyl," "C₃-C₈-cycloalkyl," "heterocycloalkyl," "aryl," "heteroaryl," "aryl C₁-C₆ alkyl", "heteroaryl C₁-C₆ alkyl," "aryl C₂-C₆ alkenyl," "heteroaryl C₂-C₆ alkenyl," "aryl C₂-C₆ alkynyl," "heteroaryl C₂-C₆ alkynyl," "C₃-C₈-cycloalkyl C₁-C₆ alkyl," or "heterocycloalkyl C₁-C₆ alkyl", and where R and R', together with the nitrogen atom to which they are attached, can optionally form a 3-8-membered heterocycloalkyl ring. Aminosulfonyl groups include cyclohexylaminosulfonyl, piperidinylsulfonyl and the like.

The term "aminosulfonyl C₁-C₆ alkyl" refers to C₁-C₆ alkyl groups having an aminosulfonyl substituent, including 2-(cyclohexylaminosulfonyl)ethyl and the like.

Unless otherwise constrained by the definition of the individual substituent, the term "substituted" refers to groups substituted with from 1 to 5 substituents selected from the group consisting of "C₁-C₆ alkyl," "C₂-C₆ alkenyl," "C₂-C₆ alkynyl," "C₃-C₈-cycloalkyl," "heterocycloalkyl," "C₁-C₆ alkyl aryl," "C₁-C₆ alkyl heteroaryl," "C₁-C₆ alkyl C₃-C₈-cycloalkyl," "C₁-C₆ alkyl heterocycloalkyl," "acyl", "amino," "amide", "aminosulfonyl," "ammonium," "acyl amino," "aminocarbonyl," "aryl," "heteroaryl," "sulfinyl," "sulfonyl," "sulphonamide", "alkoxy," "alkoxy carbonyl," "carbamate," "sulfanyl," "halogen," trihalomethyl, cyano, hydroxy, mercapto, nitro, and the like.

The term "pharmaceutically acceptable salts or complexes" refers to salts or complexes of the compounds according to the invention. Examples of such salts are formed from acid addition salts formed with inorganic acids (e.g. hydrochloric acid, hydrobromic acid, sulfuric acid, phosphoric acid, nitric acid, and the like), as well as salts formed with organic acids such as acetic acid, oxalic acid, tartaric acid, succinic acid, malic acid, fumaric acid, maleic acid, ascorbic acid, benzoic acid, tannic acid, palmoic acid, alginic acid, polyglutamic acid, naphthalene sulfonic acid, naphthalene disulfonic acid, methane sulfonic acid, p-toluene sulfonic acid and poly-galacturonic acid.

"Pharmaceutically active derivative" refers to any compound that upon administration to the recipient, is capable of providing directly or indirectly, the activity disclosed herein. The term "indirectly" also encompasses prodrugs which may be converted to the active form of the drug via endogenous enzymes or metabolism. The prodrug is a derivative of the compounds according to the invention and presenting anti-malarial activity that has a chemically or metabolically decomposable group, and a compound that may be converted into a pharmaceutically active compound according to the invention *in vivo* by solvolysis under physiological conditions. The prodrug is converted into a compound according to the present invention by a reaction with an enzyme, gastric acid or the like under a physiological condition in the living body, e.g. by oxidation, reduction, hydrolysis or the like, each of which is carried out enzymatically. These compounds can be produced from compounds of the present invention according to well-known methods.

The term "indirectly" also encompasses metabolites of compounds according to the invention. The term "metabolite" refers to all molecules derived from any of the compounds according to the present invention in a cell or organism, preferably mammal.

In the context of the present invention are encompassed pharmaceutically acceptable salts, hydrates, solvates, or polymorphs and pharmaceutically active derivatives of compounds of the invention.

The term "malaria" includes disease and conditions related to an infection by *Plasmodium.*

As used herein, "treatment" and "treating" and the like generally mean obtaining a desired pharmacological and physiological effect. The effect may be prophylactic in terms of preventing or partially preventing a disease, symptom or condition thereof and/or may be therapeutic in terms of a partial or complete cure of a disease, condition, symptom or adverse effect attributed to the disease. The term "treatment" as used herein covers any treatment of a disease in a mammal, particularly a human, and includes: (a) preventing the disease from occurring in a subject which may be predisposed to the disease but has not yet been diagnosed as having it; (b) inhibiting the disease, i.e., arresting its development; or relieving the disease, i.e., causing regression of the disease and/or its symptoms or conditions.

The term "effective amount" includes "prophylaxis-effective amount" as well as "treatment-effective amount".

The term "prophylaxis-effective amount" refers to a concentration of compound of this invention that is effective in inhibiting, decreasing the likelihood of the disease by malarial parasites, or preventing malarial infection or preventing the delayed onset of the disease by malarial parasites, when administered before infection, i.e. before, during and/or slightly after the exposure period to malarial parasites.

The term "prophylaxis" includes causal prophylaxis, i.e. antimalarial activity comprising preventing the pre-erythrocytic development of the parasite, suppressive prophylaxis, i.e. antimalarial activity comprising suppressing the development of the blood stage infection and terminal prophylaxis, i.e. antimalarial activity comprising suppressing the development of intra-hepatic stage infection. This term includes primary prophylaxis (i.e. preventing initial infection) where the antimalarial compound is administered before, during and/or after the exposure period to malarial parasites and terminal prophylaxis (i.e. to prevent relapses or delayed onset of clinical symptoms of malaria) when the antimalarial compound is administered towards the end of and/or slightly after the exposure period to malarial parasites but before the clinical symptoms. Typically, against *P. falciparum* infections, suppressive phophylaxis is used whereas against *P. vivax* or a combination of *P. falciparum* and *P. vivax,* terminal prophylaxis is used.

Likewise, the term "treatment-effective amount" refers to a concentration of compound that is effective in treating malaria infection, e.g. leads to a reduction in parasite numbers in blood following microscopic examination when administered after infection has occurred.

The term "subject" as used herein refers to mammals. For examples, mammals contemplated by the present invention include humans and the like.

### Compounds

According to one embodiment, is a provided a dispiro 1,2,4-trioxolane derivative according to Formula (I): 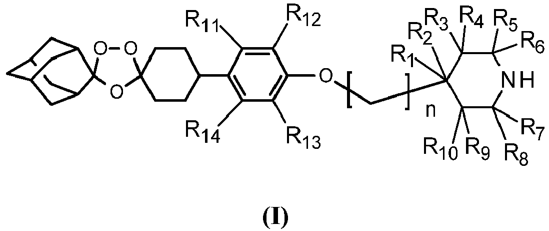 wherein R₁, R₂, R₃, R₄, R₅, R₆, R₇, R₈, R₉ and R₁₀ are independently selected from H, hydroxyl, optionally substituted C₁-C₆ alkyl such as optionally substituted methyl (e.g. methyl, trifluoromethyl) and acetamide; R₁₁, R₁₂, R₁₃ and R₁₄ are independently selected from H and halogen (e.g. Chlorine or fluorine), n is an integer selected from 1 to 3; as well as pharmaceutically acceptable salts, hydrates, solvates, or polymorphs and pharmaceutically active derivative thereof.

In a particular embodiment, the invention provides a dispiro 1,2,4-trioxolane derivative according to the invention wherein R¹ is H.

In another particular embodiment, the invention provides a dispiro 1,2,4-trioxolane derivative according to the invention wherein R² is H or hydroxy.

In a further particular embodiment, the invention provides a dispiro 1,2,4-trioxolane derivative according to the invention wherein R² is H.

In another further particular embodiment, the invention provides a dispiro 1,2,4-trioxolane derivative according to the invention wherein R² is Hydroxy.

In another particular embodiment, the invention provides a dispiro 1,2,4-trioxolane derivative according to the invention wherein R³, R⁴, R⁹ and R¹⁰ are H.

In another particular embodiment, the invention provides a dispiro 1,2,4-trioxolane derivative according to the invention wherein R₁₁, R¹², R¹³ and R¹⁴ are H.

In another particular embodiment, the invention provides a dispiro 1,2,4-trioxolane derivative according to the invention wherein R⁵, R⁶, R⁷ and R⁸ are H or optionally substituted C₁-C₆ alkyl such as optionally substituted methyl (e.g. methyl).

In a further particular embodiment, the invention provides a dispiro 1,2,4-trioxolane derivative according to the invention wherein R⁵,R⁶, R⁷and R⁸ are H.

In another further particular embodiment, the invention provides a dispiro 1,2,4-trioxolane derivative according to the invention wherein R⁵, R⁶, R⁷ and R⁸ are optionally substituted C₁-C₆ alkyl such as optionally substituted methyl (e.g. methyl).

In a particular embodiment is provided a dispiro 1,2,4-trioxolane derivative selected from the following group:
*cis*-Adamantane-2-spiro-3'-8'-[4'-[(4'-hydroxy-2',2',6',6'-tetramethyl-4'-piperidinyl) methoxy]phenyl]-1',2',4'-trioxaspiro[4.5]decane and *cis*-Adamantane-2-spiro-3'-8'-[4'-[2'-(4'-piperidinyl)ethoxy]phenyl]-1',2',4'-trioxaspiro[4.5]decane as well as pharmaceutically acceptable salts, complexes, hydrates, solvates, or polymorphs, and pharmaceutically active derivative thereof.

In a further particular embodiment is provided a trioxolane according to the invention, wherein the pharmaceutically acceptable salts are selected from mesylate and tosylate.

In a further more particular embodiment is provided a dispiro 1,2,4-trioxolane derivative selected from the following group:
*cis*-Adamantane-2-spiro-3'-8'-[4'-[(4'-hydroxy-2',2',6',6'-tetramethyl-4'-piperidinyl) methoxy]phenyl]-1',2',4'-trioxaspiro[4.5]decane *p*-tosylate and *cis*-Adamantane-2-spiro-3'-8'-[4'-[2'-(4'-piperidinyl)ethoxy]phenyl]-1',2',4'-trioxaspiro[4.5]decane mesylate; as well as pharmaceutically acceptable salts, complexes, hydrates, solvates, or polymorphs, and pharmaceutically active derivative thereof.

The dispiro 1,2,4-trioxolane derivatives of the invention are useful in the manufacture of a medicament for the prevention or treatment of malaria, are capable of killing and/or inhibiting malaria parasite replication.

According to another aspect, the dispiro 1,2,4-trioxolane derivatives of the invention are useful in the manufacture of a medicament for the prevention or treatment of cancer and schistosomiasis.

### Compositions

The invention provides pharmaceutical compositions useful for the prophylaxis or treatment of malaria. The invention further provides methods for treating a mammalian patient, and most preferably a human patient, who is suffering from malaria.

In another particular embodiment, is provided a pharmaceutical formulation containing at least one derivative according the invention and a pharmaceutically acceptable carrier, diluent or excipient thereof.

In another particular embodiment, is provided a pharmaceutical formulation comprising a compound according to Formula (I) and a further antimalarial agent as defined in the detailed description.

In another particular embodiment, is provided a pharmaceutical formulation comprising a compound according to Formula (I) and at least one further antimalarial agent selected from artemisinin and its derivatives such as artemether, artesunate, dihydroartemisinin, chloroquine, hydroxychloroquine, quinine, mefloquine, amodiaquine, atovaquone/proguanil, doxycycline, clindamycin, halofantrine, lumefantrine, pyronaridine, pyrimethamine-sulfadoxine, ferroquine, tafenoquine, piperaquine and primaquine, Spiro[3H-indole-3,1'-[1H]pyrido[3,4-b]indol]-2(1H)-one, 5,7'-dichloro-6'-fluoro-2',3',4',9'-tetrahydro-3'-methyl-,(1'R,3'S)- (CAS Registry Number: 1193314-23-6), Sulfur, [4-[[2-(1,1-difluoroethyl)-5-methyl[1,2,4]triazolo[1,5-a]pyrimidin-7-yl]amino]phenyl] pentafluoro-] (CAS Registry Number: 1282041-94-4), [3,3'-Bipyridin]-2-amine, 5-[4-(methylsulfonyl)phenyl]-6'-(trifluoromethyl)- (CAS Registry Number: 1314883-11-8) and Ethanone, 2-amino-1-[2-(4-fluorophenyl)-3-[(4-fluorophenyl)amino]-5,6-dihydroimidazo [1,2-a]pyrazin-7(8H)-yl]- (CAS Registry Number 1261109-90-3).

In another particular embodiment, is provided a pharmaceutical formulation comprising a compound according to Formula (I) and at least methylene blue.

Pharmaceutical compositions of the invention can contain one or more compound(s) of the invention in any form described herein. Compositions of this invention may further comprise one or more pharmaceutically acceptable additional ingredient(s), such as alum, stabilizers, antimicrobial agents, buffers, coloring agents, flavoring agents, adjuvants, and the like.

The compounds of the invention, together with a conventionally employed adjuvant, carrier, diluent or excipient may be placed into the form of pharmaceutical compositions and unit dosages thereof, and in such form may be employed as solids, such as tablets or filled capsules, or liquids such as solutions, suspensions, emulsions, elixirs, or capsules filled with the same, all for oral use, or in the form of sterile injectable solutions for parenteral (including subcutaneous) use. Such pharmaceutical compositions and unit dosage forms thereof may comprise ingredients in conventional proportions, with or without additional active compounds or principles, and such unit dosage forms may contain any suitable effective amount of the active ingredient commensurate with the intended dosage range to be employed. Compositions according to the invention are preferably oral.

Compositions of this invention may be liquid formulations, including, but not limited to, aqueous or oily suspensions, solutions, emulsions, syrups, and elixirs. Liquid forms suitable for oral administration may include a suitable aqueous or non-aqueous vehicle with buffers, suspending and dispensing agents, colorants, flavors and the like. The compositions may also be formulated as a dry product for reconstitution with water or other suitable vehicle before use. Such liquid preparations may contain additives, including, but not limited to, suspending agents, emulsifying agents, non-aqueous vehicles and preservatives. Suspending agents include, but are not limited to, sorbitol syrup, methyl cellulose, glucose/sugar syrup, gelatin, hydroxyethylcellulose, carboxymethyl cellulose, aluminum stearate gel, and hydrogenated edible fats. Emulsifying agents include, but are not limited to, lecithin, sorbitan monooleate, and acacia. Non-aqueous vehicles include, but are not limited to, edible oils, almond oil, fractionated coconut oil, oily esters, propylene glycol, and ethyl alcohol. Preservatives include, but are not limited to, methyl or propyl p-hydroxybenzoate and sorbic acid. Further materials as well as processing techniques and the like are set out in out in *Part 5 of* Remington's "The Science and Practice of Pharmacy'', 22nd Edition, 2012, University of the Sciences in Philadelphia, Lippincott Williams & Wilkins*,* which is incorporated herein by reference. Solid compositions of this invention may be in the form of tablets or lozenges formulated in a conventional manner. For example, tablets and capsules for oral administration may contain conventional excipients including, but not limited to, binding agents, fillers, lubricants, disintegrants and wetting agents. Binding agents include, but are not limited to, syrup, accacia, gelatin, sorbitol, tragacanth, mucilage of starch and polyvinylpyrrolidone. Fillers include, but are not limited to, lactose, sugar, microcrystalline cellulose, maizestarch, calcium phosphate, and sorbitol. Lubricants include, but are not limited to, magnesium stearate, stearic acid, talc, polyethylene glycol, and silica. Disintegrants include, but are not limited to, potato starch and sodium starch glycollate. Wetting agents include, but are not limited to, sodium lauryl sulfate. Tablets may be coated according to methods well known in the art.

Injectable compositions are typically based upon injectable sterile saline or phosphate-buffered saline or other injectable carriers known in the art.

Compositions of this invention may also be formulated as suppositories, which may contain suppository bases including, but not limited to, cocoa butter or glycerides. Compositions of this invention may also be formulated for inhalation, which may be in a form including, but not limited to, a solution, suspension, or emulsion that may be administered as a dry powder or in the form of an aerosol using a propellant, such as dichlorodifluoromethane or trichlorofluoromethane. Compositions of this invention may also be formulated transdermal formulations comprising aqueous or non-aqueous vehicles including, but not limited to, creams, ointments, lotions, pastes, medicated plaster, patch, or membrane.

Compositions of this invention may also be formulated for parenteral administration, including, but not limited to, by injection or continuous infusion. Formulations for injection may be in the form of suspensions, solutions, or emulsions in oily or aqueous vehicles, and may contain formulation agents including, but not limited to, suspending, stabilizing, and dispersing agents. The composition may also be provided in a powder form for reconstitution with a suitable vehicle including, but not limited to, sterile, pyrogen-free water.

Compositions of this invention may also be formulated as a depot preparation, which may be administered by implantation or by intramuscular injection. The compositions may be formulated with suitable polymeric or hydrophobic materials (as an emulsion in an acceptable oil, for example), ion exchange resins, or as sparingly soluble derivatives (as a sparingly soluble salt, for example).

Compositions of this invention may also be formulated as a liposome preparation. The liposome preparation can comprise liposomes which penetrate the cells of interest or the *stratum corneum*, and fuse with the cell membrane, resulting in delivery of the contents of the liposome into the cell. Other suitable formulations can employ niosomes. Niosomes are lipid vesicles similar to liposomes, with membranes consisting largely of non-ionic lipids, some forms of which are effective for transporting compounds across the *stratum corneum*.

The compounds of this invention can also be administered in sustained release forms or from sustained release drug delivery systems. A description of representative sustained release materials can also be found in the incorporated materials in *Remington's Pharmaceutical Sciences.*

### Mode of administration

Compositions of this invention may be administered in any manner, including, but not limited to, orally, parenterally, sublingually, transdermally, vaginally, rectally, transmucosally, topically, via inhalation, via buccal or intranasal administration, or combinations thereof. Parenteral administration includes, but is not limited to, intravenous, intra-arterial, intraperitoneal, subcutaneous, intramuscular, intra-thecal, and intra-articular. The compositions of this invention may also be administered in the form of an implant, which allows slow release of the compositions as well as a slow controlled i.v. infusion. In a preferred embodiment, dispiro 1,2,4-trioxolane derivatives according to the invention are administered orally.

This invention is further illustrated by the following examples that are not intended to limit the scope of the invention in any way.

The dosage administered, as single or multiple doses, to an individual will vary depending upon a variety of factors, including pharmacokinetic properties, patient conditions and characteristics (sex, age, body weight, health, size), extent of symptoms, concurrent treatments, frequency of treatment and the effect desired.

### Combination

According to the invention, the dispiro 1,2,4-trioxolane derivatives of the invention and pharmaceutical formulations thereof can be administered alone or in combination with a co-agent useful in the treatment of malaria, such as substances useful in the treatment and/or prevention of malaria e.g. for example a co-agent including, but not limited to, artemisinin and its derivatives such as arthemether, artesunate, dihydroartemisinin, chloroquine, hydroxychloroquine, quinine, mefloquine, amodiaquine, atovaquone/proguanil, doxycycline, clindamycin, halofantrine, lumefantrine, pyronaridine, pyrimethamine-sulfadoxine, ferroquine, tafenoquine, piperaquine and primaquine.

Further co-agents useful in combination with the compounds of the invention are selected from Spiro[3H-indole-3,1'-[1H]pyrido[3,4-b]indol]-2(1H)-one, 5,7'-dichloro-6'-fluoro-2',3',4',9'-tetrahydro-3'-methyl-,(1'R,3'S)- (CAS Registry Number: 1193314-23-6), Sulfur, [4-[[2-(1,1-difluoroethyl)-5-methyl[1,2,4]triazolo [1,5-a]pyrimidin-7-yl] amino]phenyl] pentafluoro-] (CAS Registry Number: 1282041-94-4), [3,3'-Bipyridin]-2-amine, 5-[4-(methylsulfonyl)phenyl]-6'-(trifluoromethyl)- (CAS Registry Number: 1314883-11-8), Ethanone, 2-amino-1-[2-(4-fluorophenyl)-3-[(4-fluorophenyl)amino]-5,6-dihydroimidazo [1,2-a]pyrazin-7(8H)-yl]- (CAS Registry Number 1261109-90-3).

The invention encompasses the administration of a dispiro 1,2,4-trioxolane derivative according to the invention or of a pharmaceutical formulation thereof, wherein the dispiro 1,2,4-trioxolane derivatives or the pharmaceutical formulation thereof is administered to an individual prior to, simultaneously or sequentially with other therapeutic regimens or co-agents useful in the treatment of malaria (e.g. multiple drug regimens), in an effective amount. Dispiro 1,2,4-trioxolane derivatives or the pharmaceutical formulations thereof that are administered simultaneously with said co-agents can be administered in the same or different composition(s) and by the same or different route(s) of administration.

### Patients

In an embodiment, patients according to the invention are patients suffering from malaria.
In another embodiment, patients according to the invention are patients with a high risk of being infected by *Plasmodium.*

In another embodiment, patients according to the invention are patients with a high risk of being infected by *Plasmodium falciparum.*

In another embodiment, patients according to the invention are patients with a high risk of being infected by *Plasmodium vivax.*

In another embodiment, patients according to the invention are patients with a high risk of being infected by *Plasmodium ovale.*

In another embodiment, patients according to the invention are patients with a high risk of being infected by *Plasmodium malariae.*

In another embodiment, patients according to the invention are patients with a high risk of being infected by *Plasmodium knowlesi.*

### Use according to the invention

In one embodiment, the invention provides a dispiro 1,2,4-trioxolane derivative according to Formula (I) as well as pharmaceutically acceptable salts, hydrates, solvates, or polymorphs, and pharmaceutically active derivative thereof for the treatment or prophylaxis of malaria.

In another embodiment, the invention provides a method for preventing or treating malaria in a subject. The method comprises administering an effective amount of an dispiro 1,2,4-trioxolane derivative according to the invention, or a pharmaceutically acceptable salt or a pharmaceutically active derivative thereof or a pharmaceutical formulation thereof in a subject in need thereof.

In another embodiment, the invention provides a use of a dispiro 1,2,4-trioxolane derivative or a method according to the invention wherein the dispiro 1,2,4-trioxolane derivative is to be administered in combination with a co-agent useful in the treatment of malaria.

In another embodiment, the invention provides a pharmaceutical composition comprising a dispiro 1,2,4-trioxolane derivative according to the invention in combination with a co-agent useful in the treatment of malaria.

In another embodiment, the invention provides a process for the preparation of an dispiro 1,2,4-trioxolane derivative according to the invention comprising the step of transforming a derivative according to Formula **(iii)** in an ether or of an intermediate of Formula **(v)** under reaction conditions for Boc deprotection to lead to a compound of Formula (I):

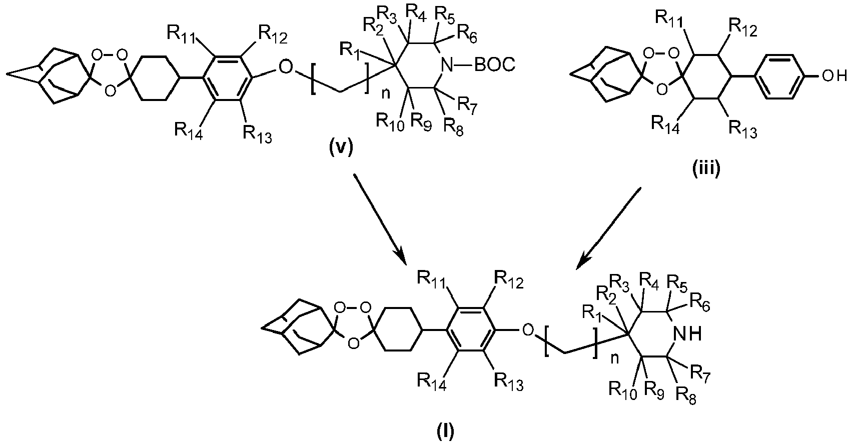

In another further embodiment, the invention provides an intermediate of Formula (v), as defined herein, in particular *cis*-adamantane-2-spiro-3'-8'-[4'-[2'-(1'-*tert*-butyloxycarbonyl -4'-piperidinyl)ethoxy]phenyl]-1',2',4'-trioxaspiro[4.5]decane.

Further, according to the earlier observed activities for dispiro 1,2,4-trioxolane *derivatives (*Hooft van Haijsduijnen et al., 2013, PLoS ONE 8(12): e82962. doi:10. 1371/journal.pone. 0082962*;* Keiser et al., 2011, Antimicrob. Agents Chemother. 2012, 56(2):1090*;* Xiao et al., 2007, Antimicrobial Agents and Chemotherapy, 1440-1445*,* Xue et al., 2014, Parasitol Res., 113:3259-3266*) the* compounds of the invention may be useful in the prevention and/or treatment of schistosomiasis or in the treatment of a cancer.

References cited herein are hereby incorporated by reference in their entirety. The present invention is not to be limited in scope by the specific embodiments described herein, which are intended as single illustrations of individual aspects of the invention, and functionally equivalent methods and components are within the scope of the invention. In the following the present invention shall be illustrated by means of some examples, which are not to be viewed as limiting the scope of the invention.

### EXAMPLES

### The following abbreviations refer respectively to the definitions below:

**g** (gram), **h** (hour), **mmol** (millimole), **mp** (melting point), **RT** (room temperature), **BOC** (tert-butyloxycarbonyl); **DMSO** (Dimethyl Sulfoxide), **MS** (Mass Spectrometry), **MHz** (Megaherz), **NMR** (Nuclear magnetic resonance), **THF** (Tetrahydrofuran), **TLC** (Thin layer chromatography).

The compounds of invention have been named according to the IUPAC standards used in the program ChemDraw Ultra (Version 12.0). The MS and NMR data provided in the examples described below are obtained as followed: Mass spectra: H¹ NMR and C¹³ NMR spectra were recorded on a 500 MHz spectrometer. The compounds were purified by precipitation and crystallization.

### Example 1: Synthesis of compounds according to the invention:

The dispiro 1,2,4-trioxolane derivative derivatives can be prepared from readily available starting materials using methods and procedures known from the skilled person. It will be appreciated that where typical or preferred experimental conditions (i.e. reaction temperatures, time, moles of reagents, solvents etc.) are given, other experimental conditions can also be used unless otherwise stated. Optimum reaction conditions may vary with the particular reactants or solvents used, but such conditions can be determined by the person skilled in the art, using routine optimisation procedures.

A general synthetic approach for obtaining compounds of Formula (I) is depicted in Scheme 1 below. Dispiro 1,2,4-trioxolane derivatives according to Formula (I), whereby the substituents are as above defined, may be prepared in one or two steps, from custom made or commercially available dispiro 1,2,4-trioxolane intermediates according to formula **(iii)** or according to formula **(iv)** following the synthetic pathways as outlined in Schemes 1 to 3 below.

According to Scheme 1, the compounds may be obtained in two steps as follows:

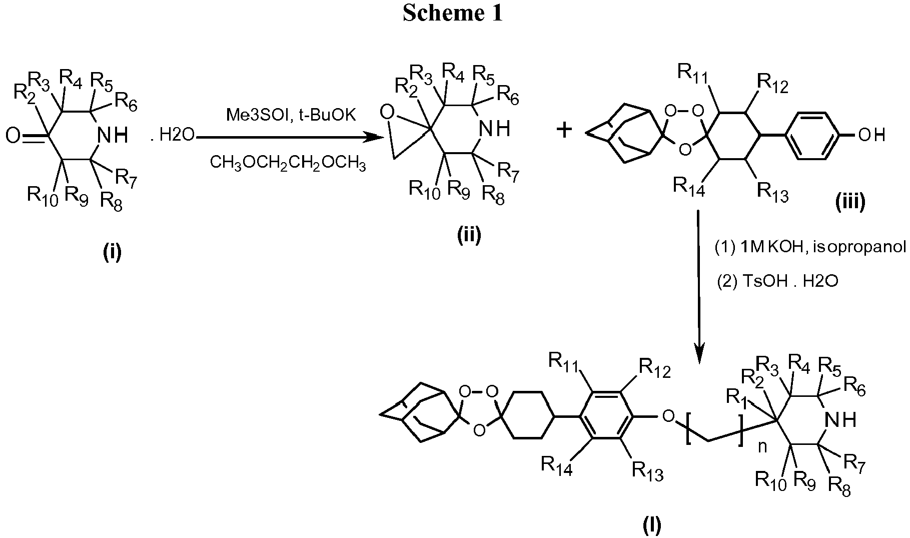

Step 1 comprises the formation of an epoxide of Formula (ii) from a ketone of formula (i) under conditions of reaction with a sulphur ylid intermediate. Step 2 comprises a step of opening of the epoxide ring of intermediate (ii) with a dispiro 1,2,4-trioxolane intermediate (iii) under conditions of reaction via phenoxide ion. Alternatively, the compounds can be obtained according to Scheme 2 below comprising a step of coupling a dispiro 1,2,4-trioxolane intermediate (iii) with an intermediate of formula (iv) under Mitsunobu coupling conditions using triphenylphosphine and an azodicarboxylate such as diisopropyl azodicarboxylate to lead to a protected intermediate of formula (v) which is then deprotected (Boc deprotection) under acidic conditions such as methanesulfonic acid in THF to lead to the formation of the compound of the invention and salts thereof:

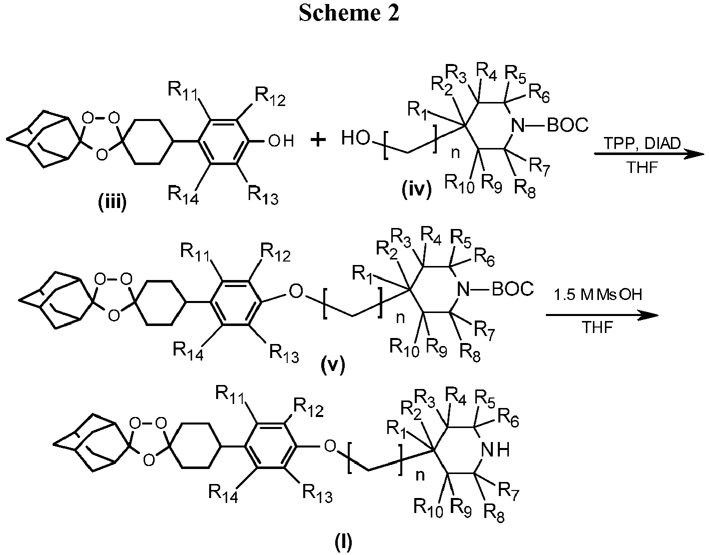

The compounds of the invention can also be obtained from intermediates of formula (iv) according to Scheme 3 below:

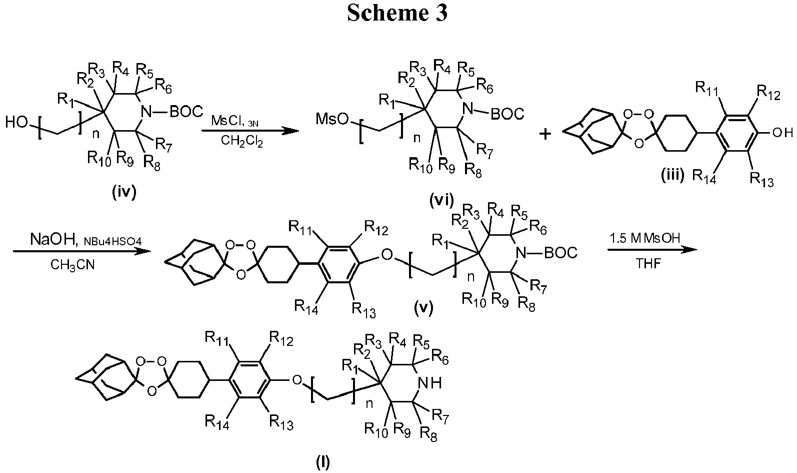

Step 1 comprises a step of the formation of a mesylate intermediate of formula (vi) under conditions using methanesulfonyl chloride which is then reacted with a dispiro 1,2,4-trioxolane intermediate (iii) in a nucleophilic substitution under basic conditions such as NaOH and tetrabutylammonium hydrogensulfate to lead to an intermediate of Formula (v) which is then deprotected (Boc deprotection) under acidic conditions such as methanesulfonic acid in THF to lead to the formation of the compound of the invention and salts thereof.

The starting material generically described in the reaction schemes which were used to synthesized compounds of the Examples are listed in Table 1 below:

**Table 1**

| **Intermediate formula** | **Chemical name** | **Structure** |
|---|---|---|
| **(i)** | 2,2,6,6,-tetramethyl-4-piperidinone monohydrate | 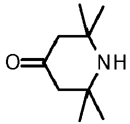 |
| **(ii)** | 5,5,7,7-tetramethyl-1-oxa-6-azaspiro[2,5]octane | 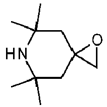 |
| **(iii)** | *cis*-adamantane-2-spiro-3'-8'-(4'-hydroxyphenyl)-1',2',4'-trioxaspiro[4.5]decane | 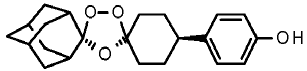 |
| **(iv)** | tert-butyl 4-(2-hydroxy-ethyl)piperidine-1-carboxylate | 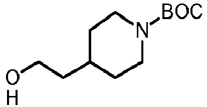 |
| **(v)** | c*is-*adamantane-2-spiro-3'-8'-[4'-[2'-(1'-*tert*-butyloxycarbonyl -4'-piperidinyl)ethoxy]phenyl]-1',2',4'-trioxaspiro[4.5]decane | 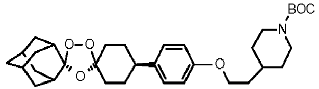 |
| **(vi)** | tert-butyl 4-(2-methylsulfonyloxy-ethyl)piperidine-1-carboxylate | 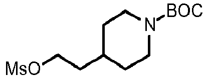 |

### Compound (1)

*cis*-Adamantane-2-spiro-3'-8'-[4'-[(4'-hydroxy-2',2',6',6'-tetramethyl-4'-piperidinyl) methoxy]phenyl]-1',2',4'-trioxaspiro[4.5]decane (compound of Formula (1) wherein R¹, R³, R⁴, R⁹, R¹⁰ and R¹¹ are H, R² is hydroxyl, R⁵, R⁶, R⁷ and R⁸ and R¹¹ are methyl and n is 1) was synthesized according to Scheme 1 as described in Scheme 1a:

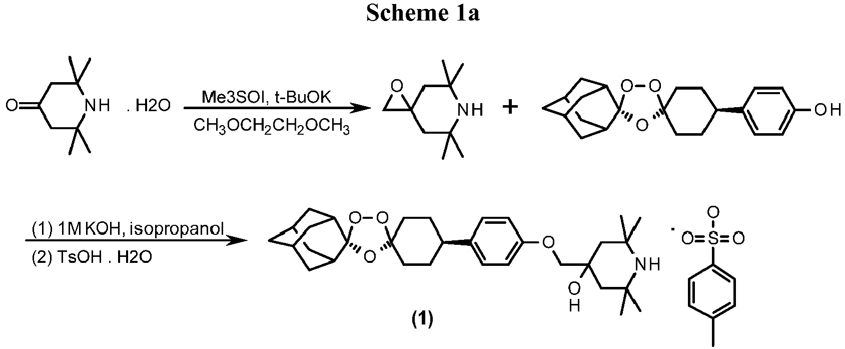

**Step 1:** An intermediate of formula **(ii)** is prepared as described in Cygler et al., 1986, J. Chem., 64(4), 670-680*.* A mixture of an intermediate **(i)** 2,2,6,6,-tetramethyl-4-piperidinone monohydrate (3.46 g, 20.0 mmol), trimethylsulfoxonium iodide (4.4 g, 20.0 mmol), potassium t-butoxide (4.4 g, 40.0 mmol) and ethylene glycol dimethyl ether (80 ml) was refluxed for 8 h with good stirring under a N₂ atmosphere. After cooling to room temperature, the reaction mixture was concentrated to 50 ml and diluted with ether (300 ml), washed with water (300 ml) and brine (300 ml), dried over MgSO₄, filtered and concentrated to afford an intermediate of formula **(ii),** 5,5,7,7-tetramethyl-1-oxa-6-azaspiro[2,5]octane, as color less oil (3.0 g, 89%). ¹HNMR (500 MHz, CDCl₃): δ 1.21 (s, 6H), 1.25 (s, 6H), 1.48 (q, J=7.5Hz, 4H), 2.69 (s, 2H).

**Step 2:** To a solution of the intermediate of formula **(iii)** *cis*-adamantane-2-spiro-3'-8'-(4'-hydroxyphenyl)-1',2',4'-trioxaspiro[4.5]decane (0.5 g, 1.40 mmol) and the intermediate of formula **(ii),** 5,5,7,7-tetramethyl-1-oxa-6-azaspiro[2,5]octane (0.50 g, 2.96 mmol) in isopropanol (25 ml) was added 1M KOH aqueous solution (4 ml). The resulting mixture was stirred at 60 °C for 20 h and then quenched with water (20 ml). The precipitate was collected by filtration and washed with water to afford a mixture of intermediate of formula **(iii)** and compound **(1)** as a free base (0.70 g, ~1:1). The crude product was dissolved in ether (10 ml) and then the solution of p-toluene sulfonic acid monohydrate (0.15 g) in ethyl acetate (30 ml) was added. The precipitate was collected by filtration to afford compound **(1)** as a p-tosylate salt as a colorless solid (0.47 g, 48%). mp 153-154°C; ¹HNMR (500 MHz, CDCl₃): δ 1.54-2.08 (m, 38H), 2.26 (s,3H), 2.45-2.54 (m, 1H), 2.49 (s, 1H), 3.52 (s, 2H), 6.73 (d, J=8.5Hz, 2H), 7.07-7.16 (m, 4H), 7.55 (br, d, J=12.0Hz, 1H), 7.73 (d, J=8.0Hz, 2H), 8.79 (br, d, J= 12.0Hz, 1H); ¹³CNMR (125.7 MHz, CDCl₃): δ 21.24, 25.84, 26.45, 26.85, 31.21, 31.62, 34.69, 34.78, 36.37, 36.77, 40.93, 42.03, 44.11 (br), 56.29, 70.39, 108.37, 111.40, 114.46, 125.84, 127.70, 128.88, 139.27, 140.26, 142.31, 156.41.

### Compound 2

*cis*-Adamantane-2-spiro-3'-8'-[4'-[2'-(4'-piperidinyl)ethoxy]phenyl]-1',2',4'-trioxaspiro [4.5]decane (compound of Formula (**I**) wherein R¹ to R¹¹ are H and n is 2) was synthesized according to Scheme 2 as described in Scheme 2a:

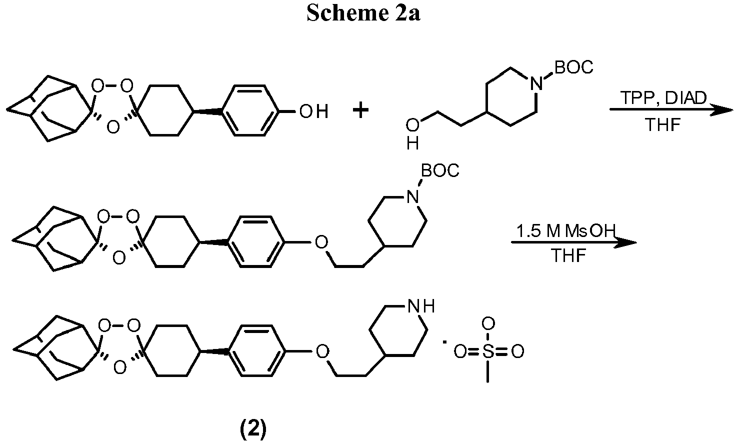

**Step 1:** Diisopropyl azodicarboxylate (0.31 ml, 1.53 mmol) was added dropwise to a mixture of an intermediate of formula **(iii)** *cis*-adamantane-2-spiro-3'-8'-(4'-hydroxyphenyl)-1',2',4'-trioxaspiro[4.5]decane (0.50 g, 1.40 mmol), tert-butyl 4-(2-hydroxyethyl)piperidine-1-carboxylate (0.32 g, 1.40 mmol), triphenylphosphine (0.40 g, 1.53 mmol) in THF (20 ml) at 0°C under N₂ atmosphere. The resulting mixture was stirred at rt. for 24 h. After removal of solvents, the crude product was purified by crystallization from EtOH to afford desired BOC intermediate of formula **(iv),** *cis*-adamantane-2-spiro-3'-8'-[4'-[2'-(1'-tert-butyloxycarbonyl-4'-piperidinyl)ethoxy] phenyl]-1',2',4'-trioxaspiro[4.5]decane, as colorless solid (0.31 g, 39%). ¹HNMR (500 MHz, CDCl₃): δ 1.10-1.22 (m, 2H), 1.45 (s, 9H), 1.64-2.08 (m, 29H), 2.45-2.54 (m, 1H), 2.60-2.76 (m, 2H), 3.98 (t, J= 6.0Hz, 2H), 4.00-4.20 (m, 2H), 6.81 (d, J=8.5Hz, 2H), 7.11 (d, J=8.5Hz, 2H); ¹³CNMR (125.7 MHz, CDCl₃): δ 26.65, 27.05, 28.62, 31.81, 32.23, 33.11, 34.91, 34.97, 35.98, 36.57, 36.97, 44.11 (br), 42.21, 65.44, 79.38, 108.59, 111.52, 114.47, 127.76, 138.50, 155.02, 157.45.

**Step 2:** A mixture of the intermediate of formula **(iv),** *cis*-adamantane-2-spiro-3'-8'-[4'-[2'-(1'-*tert*-butyloxycarbonyl-4'-piperidinyl)ethoxy]phenyl]-1',2',4'-trioxaspiro[4.5] decane (0.30 g, 0.53 mmol) and 1.5M MsOH in THF (10 ml) was stirred at room temperature for 4 h. The resulting precipitate was filtered off, and washed with ether (30 ml) and dried *in vacuo* to afford compound (**2**) as a mesylate salt as colorless solid (0.22 g, 73%). mp 139-140°C; ¹HNMR (500 MHz, CDCl₃): δ 1.56-2.08 (m, 29H), 2.45-2.54 (m, 1H), 2.78 (s, 3H), 2.82-2.94 (m, 2H), 3.44-3.53 (m, 2H), 3.97 (t, J= 6.0Hz, 2H), 6.80 (d, J=8.5Hz, 2H), 7.11 (d, J=8.5Hz, 2H), 8.41 (br, s, 1H), 8.76 (br, s, 1H); ¹³CNMR (125.7 MHz, CDCl₃): δ 16.94, 26.46, 26.86, 28.62, 31.05, 31.63, 34.72, 34.79, 35.11, 36.38, 36.78, 39.29, 42.02, 44.19, 64.59, 108.41, 111.38, 114.23, 127.67, 138.59, 157.00.

Alternatively, *cis*-Adamantane-2-spiro-3'-8'-[4'-[2'-(4'-piperidinyl)ethoxy]phenyl]-1',2',4'-trioxaspiro[4.5]decane ] was synthesized according to Scheme 3 as described in Scheme 3a:

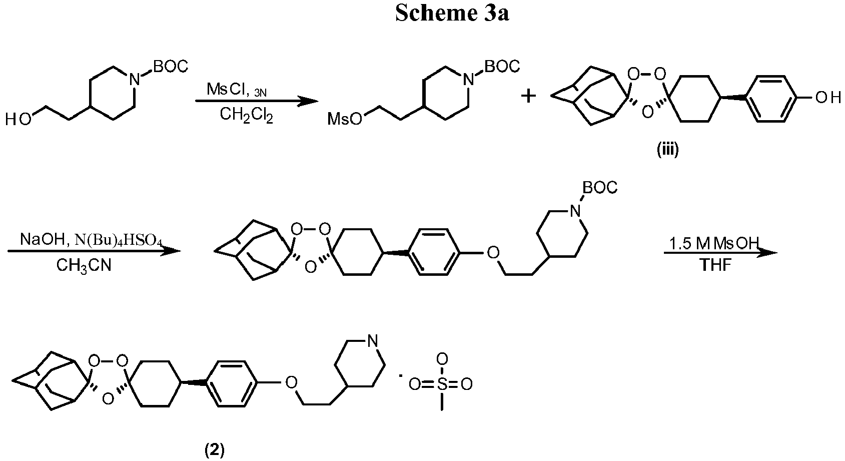

**Step 1:** To a solution of tert-butyl 4-(2-hydroxyethyl)piperidine-1-carboxylate (1.00 g, 4.36 mmol) as intermediate of formula **(iv)** and triethylamine (1.00 ml, 8.00 mmol) in CH₂Cl₂ (20 ml) at 0°C was added drop wise methanesulfonyl chloride (0.41 ml, 5.24 mmol). After being stirred at 0°C for 1 h and at rt overnight, the reaction mixture was washed with water (2 x 10 ml) and brine (10 ml), dried over MgSO₄ filtered and concentrated to afford an intermediate of formula **(vi)** tert-butyl 4-(2- methylsulfonyloxy-ethyl)piperidine-1-carboxylate as a pale yellow solid (1.25 g, 93%). ¹HNMR (500 MHz, CDCl₃): δ 1.08-1.20 (m, 2H), 1.46 (s, 9H), 1.56-1.76 (m, 7H), 2.64-2.76 (m, 2H), 4.04-4.16 (m, 2H), 4.29 (t, J= 6.0Hz, 2H).

**Step 2:** To a solution of an intermediate of formula **(iii)** *cis*-adamantane-2-spiro-3'-8'-(4'-hydroxyphenyl)-1',2',4'-trioxaspiro[4.5]decane (0.70 g, 1.97 mmol) in dry acetonitrile (80 ml) were added powered NaOH (0.50 g, 12.5 mmol) and tetrabutylammonium hydrogensulfate (0.05 g, 0.15 mmol). The mixture was stirred at rt for 30 min before the intermediate of formula **(vi)** tert-butyl 4-(2- methylsulfonyloxy-ethyl)piperidine-1-carboxylate (0.61 g, 1.98 mmol) was added. The reaction mixture was stirred at 60°C overnight, cooled to rt, filtered, and washed with CH₂Cl₂. After the filtrate was concentrated, the crude product was purified by crystallization from EtOH/H₂O (1:1) to afford intermediate of formula (v), *cis-*adamantane-2-spiro-3'-8'-[4'-[2'-(1'-*tert*-butyloxycarbonyl-4'-piperidinyl)ethoxy]phenyl]-1',2',4'-trioxaspiro[4.5] decane, as colorless solid (0.90 g, 81%). ¹HNMR (500 MHz, CDCl₃): δ 1.10-1.22 (m, 2H), 1.45 (s, 9H), 1.64-2.08 (m, 29H), 2.45-2.54 (m, 1H), 2.60-2.76 (m, 2H), 3.98 (t, J= 6.0Hz, 2H), 4.00-4.20 (m, 2H), 6.81 (d, J=8.5Hz, 2H), 7.11 (d, J=8.5Hz, 2H); ¹³CNMR (125.7 MHz, CDCl₃): δ 26.65, 27.05, 28.62, 31.81, 32.23, 33.11, 34.91, 34.97, 35.98, 36.57, 36.97, 42.21, 65.44, 79.38, 108.59, 111.52, 114.47, 127.76, 138.50, 155.02, 157.45

**Step 3:** A mixture of the BOC intermediate of formula **(v)** (0.90 g, 1.59 mmol) and 1.5M MsOH in THF (30 ml) was stirred at room temperature for 4 h. The resulting precipitate was filtered off, and washed with ether (30 ml) and dried *in vacuo* to afford compound **(2)** as a mesylate salt, as colorless solid (0.70 g, 78%). mp 139-140°C; ¹HNMR (500 MHz, CDCl₃): δ 1.56-2.08 (m, 29H), 2.45-2.54 (m, 1H), 2.78 (s, 3H), 2.82-2.94 (m, 2H), 3.44-3.53 (m, 2H), 3.97 (t, J= 6.0Hz, 2H), 6.80 (d, J=8.5Hz, 2H), 7.11 (d, J=8.5Hz, 2H), 8.41 (br, s, 1H), 8.76 (br, s, 1H); ¹³CNMR (125.7 MHz, CDCl₃): δ 16.94, 26.46, 26.86, 28.62, 31.05, 31.63, 34.72, 34.79, 35.11, 36.38, 36.78, 39.29, 42.02, 44.19, 64.59, 108.41, 111.38, 114.23, 127.67, 138.59, 157.00.

If the above synthetic methods are not applicable to obtain dispiro 1,2,4-trioxolane derivatives according to the invention and/or necessary intermediates, suitable methods of preparation known by a person skilled in the art should be used. In general, the synthesis pathways for any individual derivative will depend on the specific substituents of each molecule and upon the ready availability of intermediates necessary; again such factors being appreciated by those of ordinary skill in the art. For all the protection and deprotection methods, see Philip J. Kocienski, in "Protecting Groups", Georg Thieme Verlag Stuttgart, 3rd Edition, 2005 and Theodora W. Greene and Peter G. M. Wuts in "Protective Groups in Organic Synthesis ", Wiley Interscience, 5th Edition 2014*.* Compounds of this invention can be isolated in association with solvent molecules by crystallization from evaporation of an appropriate solvent. The pharmaceutically acceptable acid addition salts of the dispiro 1,2,4-trioxolane derivatives, may be prepared in a conventional manner. For example, a solution of the free base may be treated with a suitable acid, either neat or in a suitable solution, and the resulting salt isolated either by filtration or by evaporation under vacuum of the reaction solvent. Pharmaceutically acceptable base addition salts may be obtained in an analogous manner by treating a solution of a dispiro 1,2,4-trioxolane derivative with a suitable base. Both types of salts may be formed or interconverted using ion-exchange resin techniques.

### Example 2: Method used to assess the solubility of compounds of the invention

The solubility of the compounds of the invention was assessed in comparison from another dispiro 1,2,4-trioxolane described in WO 09/091433 of the following formula (comparative compound C1):

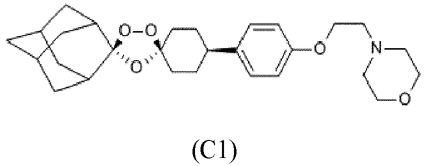

Compounds were each accurately weighed into individual screw cap polypropylene tubes and media added to give a nominal minimum target compound concentration of 2.2 mg/mL. Samples were vortex mixed and placed in a 37°C incubator where they were continually mixed on an orbital shaker (IKA® VXR basic Vibrax® orbital shaker) at 600 rpm. Incubation times were 1 hour for fasted state simulated gastric fluid (FaSSGF) and 5 hours for fasted and fed state simulated intestinal fluids (FaSSIF-V2 and FeSSIF-V2 respectively). Sampling was carried out by centrifuging each sample at 10,000 rpm for 3 minutes, transferring 300 µL aliquots into fresh Eppendorf tubes and centrifuging these tubes again at 10,000 rpm for 3 minutes. Triplicate aliquots of each final supernatant were diluted 20-fold in 50% aqueous methanol, and then further diluted in 50% aqueous acetonitrile to be within an appropriate concentration range for analysis by LC-MS. LC-MS methods and validation data for the analysis of solubility samples are shown in Table 2 below and the media composition is shown in Table 3.

**Table 2**

| **HPLC Conditions** | | | | | | | | | | |
|---|---|---|---|---|---|---|---|---|---|---|
| **Instrument** | Micromass Xevo TQ coupled to a Waters Acquity UPLC | | | | | | | | | |
| **Detection** | Positive electrospray ionisation multiple-reaction monitoring mode | | | | | | | | | |
| **Column** | Ascents Express Amide column (50 x 2.1 mm, 2.7 µm) | | | | | | | | | |
| **LC conditions** | Gradient cycle time: 4 min; Injection vol: 3 µL; Flow rate: 0.4 mL/min | | | | | | | | | |
| **Mobile phase** | acetonitrile-water with 0.05% formic acid | | | | | | | | | |

| | **Time (min)** | **Water containing 0.05% formic acid (%)** | | | | **Acetonitrile containing 0.05% formic acid (%)** | | | | |
|---|---|---|---|---|---|---|---|---|---|---|
| | | Compounds 2 & 3 | | C1 | | Compounds 2 & 3 | | C1 | | |
| **Gradient** | 0 | 98 | | 99.9 | | 2 | | 0.1 | | |
| | 0.2 | 98 | | 99.9 | | 2 | | 0.1 | | |
| | 0.3 | 95 | | 98 | | 5 | | 2 | | |
| | 2.7 | 5 | | 5 | | 95 | | 95 | | |
| | 2.8 | 5 | | 5 | | 95 | | 95 | | |
| | 3.3 | 5 | | 5 | | 95 | | 95 | | |
| | 3.5 | 98 | | 99.9 | | 2 | | 0.1 | | |
| | 4.0 | 98 | | 99.9 | | 2 | | 0.1 | | |

| **Mass Spectrometer Conditions** | | | | | | | | | | |
|---|---|---|---|---|---|---|---|---|---|---|
| **Analyte** | **tR** (min) | **Transition** (*m*/*z*) | | | | **Cone voltage** (V) | | **CID** (V) | | |
| (1) | 2.2 | 468.38 > 302.29 | | | | 35 | | 30 | | |
| (2) | 2.2 | 526.42 > 360.26 | | | | 35 | | 40 | | |
| C1 | 2.2 | 470.21 > 303.88 | | | | 35 | | 35 | | |
| Diazepam | 2.0 | 285.12 > 154.09 | | | | 50 | | 25 | | |

| **Validation Data** | | | | | | | | | | |
|---|---|---|---|---|---|---|---|---|---|---|
| | **QC Data** | | | | | | **Calibration Data ^{c}** | | | |
| **Compound** | **QC conc (ng/mL)** | | **Accuracy a (% bias)** | | **Precision (%RSD)** | | **Range (ng/mL)** | | **R2** | **LLQ**^{b} (ng/m L) |
| (1) | 500 (n=6) | | -1.4 | | 2.2 | | 2.5 - 5,000 | | 0.9999 | 2.5 |
| (2) | 500 (n=6) | | 4.6 | | 3.0 | | 2.5 - 2,500 | | 0.9994 | 2.5 |
| C1 | 500 (n=6) | | -6.6 | | 5.1 | | 2.5 - 2,500 | | 0.9999 | 2.5 |

| | | | | | | | | | | |
|---|---|---|---|---|---|---|---|---|---|---|
| *^{a}Acceptance criteria for batch analysis: at least 67% of the QC samples lie within ± 15% of nominal values; blower limit of quantitation (LLQ) is defined by the lowest acceptable calibration standard for which the back calculated concentration lies within ± 20% of the nominal concentration; ^{c}50% acetonitrile-water was used for preparation of the calibration standard and QC samples. Data were fitted to linear (C1) or non-linear equations ((1) and (2)).* | | | | | | | | | | |

**Table 3**

| **Medium composition** | **Concentration** |
|---|---|
| ***Fasted State Simulated Gastric Fluid* (*FaSSGF*) *without pepsin* *** | |
| Sodium Taurocholate | 80 uM |
| Lecithin | 20 µM |
| Sodium Chloride | 34.2 mM |
| HCl | Sufficient to achieve pH 1.6 |
| Milli-Q Water | - |
| Final pH | 1.6 |
| **Fasted State Simulated Intestinal Fluid- Version 2 (FaSSIF-V2)*** | |
| Sodium Taurocholate | 3 mM |
| Lecithin | 0.2 mM |
| Sodium Hvdroxide | 34.8 mM |
| Sodium Chloride | 68.6 mM |
| Maleic Acid | 19.1 mM |
| Milli-Q Water | - |
| Final pH | 6.5 |
| ***Fed State Simulated Intestinal Fluid-Version 2* (*FeSSIF-V2*)***** | |
| Sodium Taurocholate | 10 mM |
| Lecithin | 2.0 mM |
| Glycerol Monooleate | 5.0 mM |
| Sodium Oleate | 0.8 mM |
| Sodium Hvdroxide | 81.7 mM |
| Sodium Chloride | 125.5 mM |
| Maleic Acid | 55.0 mM |
| Milli-Q Water | - |
| Final pH | 5.8 |

| | |
|---|---|
| *** Jantratid et al. 2008, Pharm. Res., 25, 1663-1676 | |

The solubility data in-biorelevant media are presented under Table 4 below:

**Table 4**

| **Compound** | **Media** | **Conc. (µg/mL)^{a}** | **Final pH** |
|---|---|---|---|
| 1 | FaSSGF | 10.1 | 1.5 |
| | FaSSIF-V2 | 1516 | 6.7 |
| | FeSSIF-V2 | >2000 | 5.9 |
| | PBS, pH 7.4 | 10.6 | 7.4 |
| 2 | FaSSGF | 5.6 | 1.5 |
| | FaSSIF-V2 | 293 | 6.5 |
| | FeSSIF-V2 | 1632 | 5.8 |
| | PBS, pH 7.4 | <1^{c} | 7.4 |
| Comparative (C1) | FaSSGF | 51.9⁶/79.0 | 1.5/1.7 |
| | FaSSIF-V2 | 22.3/40.6 | 6.2/6.0 |
| | FeSSIF-V2 | 557/563 | 5.8/5.8 |
| | PBS, pH 7.4 | <1^{c}/ 0.085 | 7.3/7.3 |

| | | | |
|---|---|---|---|
| *^{a}Data represents the mean of triplicate measurements unless stated otherwise. ^{b} Value calculated from duplicate measurement.^{c} Samples below lower limit of quantitation (LLQ) (2.5 ng*/*mL) at 400 x dilution factor.* | | | |

Those results support that for compounds of the invention, FaSSIF solubility values are much higher than for comparative compound C1. Solubility at pH = 2 in chloride-containing media is also much higher for compounds of the invention than comparative compound C1.

### Example 3: Time-dependent CYP inhibition

Time-dependent CYP inhibition can occur as a result of irreversible interactions of a test compound with a CYP450 enzyme (i.e. mechanism-based inhibition; MBI) or, alternatively, by reversible product inhibition (i.e. whereby the metabolite of the test compound is responsible for inhibition of the CYP). The potential for time-dependent CYP inhibition was assessed using CYP-specific metabolic pathways in human liver microsomes by measuring the formation of metabolites known to be formed by individual CYP isoforms (Table 5) according to the following protocol (probe substrates, metabolites and incubation times):

**Table 5**

| **CYP isoform** | **Substrate (conc)^{a}** | **Specific Metabolite** | **Incubation Time (min)** | **Control Mechanis m-Based Inhibitor** | **Control Reversible Inhibitor** |
|---|---|---|---|---|---|
| 1A2 | phenacetin (400 µM) | paracetamol | 30 | furafylline | Ketoconazole |
| 2B6 | bupropion (750 µM) | hydroxybupropion | 20 | ticlopidine | Ketoconazole |
| 2C8 | amodiaquine (25 µM) | N-desethylamodiaquin e | 10 | gemfibrozil glucuronide | quercetin |
| 2C9 | tolbutamide (2'100 µM) | hydroxytolbutamide | 30 | tienilic acid | Sulfaphenazole |
| 2C19 | (S)-mephenytoin (200 µM) | 4'-hydroxymephenytoi n | 40 | (S)-fluoxetine | Ketoconazole |
| 2D6 | dextromethor phan (15 µM) | dextrorphan | 15 | paroxetine | quinidine |
| 3A4/5 | midazolam (7.5 µM) | 1'-hydroxymidazolam | 10 | verapamil | Ketoconazole |
| | testosterone (120 µM) | 6β-hydroxytestosterone | 10 | verapamil | Ketoconazole |

| | | | | | |
|---|---|---|---|---|---|
| *^{a}Substrate concentrations used were approximately 5xKm for each metabolic pathway ^{b} Recommended that two structurally unrelated substrates be used for CYP3A;* Bjornsson et al., 2003, Drug Metab. Dispos., 31: 815-832*. Note: Midazolam 1'-hydroxylation and testosterone 6β-hydroxylation are not specific to the CYP3A4 isoform, but can also be mediated by CYP3A5, The potential for inhibition of CYP3A5 cannot be ruled out on the basis of these results (*Huang et al., 2004, Drug Metab. Dispos, 32: 1434-1445*).* | | | | | |

The assessment of time-dependent inhibition was based on measurement of the "IC50 shift", whereby the IC₅₀ was measured following pre-incubation of test compound (or reference inhibitor) in the absence or presence of NADPH. A 10-fold dilution step was incorporated following the pre-incubation to minimize reversible product inhibition (by potential metabolites of test compound) such that evidence of time-dependent inhibition is more likely to reflect mechanism-based inhibition. The maximum concentration of test compound in the pre-incubation mixture (containing either 1 or 2 mg/mL microsomal protein and 1.0% v/v DMSO) was 200 µM, and the potential for precipitation of test compound was closely monitored at the time of spiking. For each compound, a precipitate was observed immediately after compound was added to the pre-incubation mixture, however this precipitate disappeared following mixing and equilibration at 37°C for approximately 40 min. On this basis, it is assumed that test compounds were fully dissolved at all concentrations in the pre-incubation mixtures. Reference inhibitors (both mechanism-based and reversible inhibitors) were included to validate the assay.

***Pre-incubation**:* Multiple concentrations of test compound or reference inhibitor were incubated in the absence and presence of NADPH at 37°C with human liver microsomes suspended in 0.1 M phosphate buffer (pH 7.4) for 30 min at 10-fold the final assay concentration. The microsomal protein concentration during the pre-incubation period was 1 mg/mL (CYPs 1A2, 2A6, 2B6, 2C8, 2D6, 2E1 and 3A4) and 2 mg/mL (CYPs 2C9 and 2C19), and the total concentration of organic solvent in the pre-incubation mixture was 1% (v/v).

***Assay:*** Following the 30 min pre-incubation, aliquots of the pre-incubation mixture were diluted 10-fold in 0.1 M phosphate buffer (pH 7.4) containing the respective probe substrate (contributing 0.2% organic solvent to the final mixture) for an individual CYP isoform (Table 6). An NADPH-regenerating buffer system was added to all samples which were then incubated at 37°C for 10 - 40 min. The total concentration of organic solvent in the final incubation mixture was 0.3% (v/v).

**Table 6**

| **Activity** | **C1** | **(2)** | **(1)** |
|---|---|---|---|
| **CLᵢₙₜ (µL/min/mg) H, D, R, M** | 69, 18, 14, 10 | <7,9, <7,16 | 14, <7, <7, <7 |
| **Cyp Inhibition IC₅₀ (µM)** | 14.4 µM (3A4-Tes) | >20 µM 7 isoforms | >20 µM 7 isoforms |
| **Time Dependent Inhibition** | Positive for 3A4 | none | Weak on 3A4 |

These data show that compounds of the invention have low clearance and do not exhibit CYP inhibition out to beyond 20 micromolar, nor time dependent inhibition.

### Example 4 : Anti-malarial in vitro and in vivo efficacy of compounds according to the invention

The ability of dispiro 1,2,4-trioxolane derivatives according to the invention to show antimalarial efficacy *in vitro and in vivo* was tested as described in Charman, et al, 2011, PNAS, 108, 4400-4405 against chloroquine-resistant (K1) and chloroquine-sensitive (NF54) strains of *P*. *falciparum in vitro* (Vennerstrom et al. 2004, Nature, 430, 900-904*).* For studies with synchronized parasites, stages were incubated for 24 h with comparative compound (C1), washed, and then incubated with hypoxanthine, as previously described (Hofer et al. 2008, J Antimicrob Chemother, 62, 1061-1064*).*

Table 7 below shows the *in vitro* IC₅₀s (ng/ml) against different strains of *P. falciparum,* namely K1 and NF54. The *in vivo* activity is shown in P. *berghei* (Pb) infected mice in terms of the percentage of inhibition of parasitemia following compound administration and the number of survival days.

**Table 7**

| **Activity** | **C1** | **(1)** | **(2)** |
|---|---|---|---|
| **IC₅₀ (ng/mL)** **K1** **NF54** | 1.5 2.0 | 1.4 1.4 | 1.4 1.4 |
| **Pb 1 x 30 mg/kg** **% activity** **Survival days** | 98.1 >30 | 99.3 >30 | 99.5 >30 |
| **Pb 1 x 10 mg/kg** **% activity** **Survival days** | 99.5 15.2 | 99.1 13.6 | 99.4 13.4 |

These data show that compounds of the invention are able to inhibit parasite proliferation in a similar manner to a compound in clinical development.

### Example 5: Anti-malarial activity on artemisinin-resistant P. falciparum isolate

Further, compounds of the invention were further tested on artemisinin-resistant *P*. *falciparum* isolate from Cambodia and compared to compound C1 and Dihydroartemisinin (DHA) according to the following Ring-stage survival assays (RSA₀₋₃ₕ), essentially as previously described by Witkowski et al., 2013, Antimicrob. Agents Chemother. 57:914-93)*,* but with a few modifications in the drug washing procedure to ensure that no residual peroxide was present during the 66h post-treatment period. Briefly, 0-3 h post-invasion ring stages were adjusted to 1% parasitemia and 2.5% haematocrit by adding uninfected erythrocytes, transferred in a total volume of 1 mL into 48-well plates and exposed for 6 h to a range of concentrations (700, 350, 175, 88 and 49 nM) of DHA or one of the compounds tested in this study. After 6 h, cultures were transferred to 15 mL conical tubes, centrifuged at 1400 rpm (400g) for 2 min and carefully washed 2 times with 12 mL of culture medium. The complete removal of the compound after washing was verified by incubating the supernatant recovered after the last washing step with fresh cultures of NF54 parasites and ensuring that no growth inhibition was detected. After washing, blood pellets were resuspended in complete compound-free culture medium, transferred into new wells and cultured for 66 h in standard conditions. Table 8 below presents the Ring-stage survival assay (RSA) values as tested *in Wells et al., 2015, supra* for compounds of the invention and comparative compounds.

**Table 8**

| **Compounds** | **RSA values ± SEM at different concentrations in nM** | | | | |
|---|---|---|---|---|---|
| | 700 nM | 350 nM | 175 nM | 88 nM | 49 nM |
| DHA | 33.02 ±4.39 | 37.67 ± 3.65 | 47.94 ±3.88 | 60.03 ± 4.99 | 73.5 ± 12.3 |

| **Compounds** | **RSA values ± SEM at different concentrations in nM** | | | | |
|---|---|---|---|---|---|
| (C1) | 0.73 ±1.03 | 0.73 ±1.03 | 0.73 ±1.03 | 0.92 ±0.92 | 13.7 ± 1.2 |
| (1) | 0 | 0 | 0.11 ±0.08 | 0.16 ± 0.23 | 1.29 ± 1.24 |
| (2) | 0 | 0 | 0 | 0 | 0.76 ± 0.60 |

It was observed that DHA led survival ranging from 74 to 33% (at 49 and 700 nM, respectively), which is comparable with the observed values from Straimer et al., 2016, Trends in Parasitology, 32, 682-696*).* C1 tested at 700 nM was about 45 times more potent than DHA.

Compound (2) is completely inhibiting the growth of the artemisinin-resistant isolate at the 700, 350, 175 and 88 nM and Compound (1) is more than 5 fold more potent than C1. Assuming that RSA0-3h can indeed predict the potency of compounds against artemisinin-resistant parasites in man, those data would suggest that compounds of the invention are highly potent against this tested artemisinin-resistant strain.

## Claims

1. A dispiro 1,2,4-trioxolane according to Formula (I): 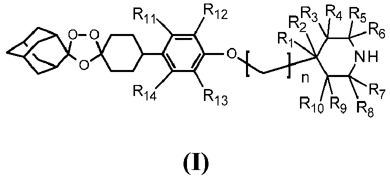 wherein R₁, R₂, R₃, R₄, R₅, R₆, R₇, R₈, R₉ and R₁₀ are independently selected from H, hydroxyl, optionally substituted C₁-C₆ alkyl and acetamide; R₁₁, R₁₂, R₁₃ and R₁₄ are independently selected from H and halogen, n is an integer selected from 1 to 3; as well as pharmaceutically acceptable salts, hydrates, solvates, or polymorphs and pharmaceutically active derivative thereof.

2. A trioxolane according to claim 1 wherein R₁ is H.

3. A trioxolane according to claim 1 or 2 wherein R₂ is H or hydroxy.

4. A trioxolane according to claim 1 or 2 wherein R₂ is H.

5. A trioxolane according to claim 1 or 2 wherein R₂ is hydroxy.

6. A trioxolane according to any one of claims 1 to 5 wherein R₃, R₄, R₉ and R₁₀ are H.

7. A trioxolane according to any one of claims 1 to 6 wherein R₅, R₆, R⁷ and R⁸ are H or optionally substituted C₁-C₆ alkyl such as optionally substituted methyl.

8. A trioxolane according to any one of claims 1 to 6 wherein R₅, R₆, R₇ and R₈ are H.

9. A trioxolane according to any one of claims 1 to 6 wherein R₅, R₆, R₇ and R₈ are optionally substituted C₁-C₆ alkyl.

10. A trioxolane according to any one of claims 1 to 9 wherein R₁₁, R₁₂, R₁₃ and R₁₄ are H.

11. A trioxolane according to any one of claims 1 to 10 selected from the following group:
*cis*-Adamantane-2-spiro-3'-8'-[4'-[(4'-hydroxy-2',2',6',6'-tetramethyl-4'-piperidinyl)methoxy]phenyl]-1',2',4'-trioxaspiro[4.5]decane and *cis*-Adamantane-2-spiro-3'-8'-[4'-[2'-(4'-piperidinyl)ethoxy]phenyl]-1',2',4'-trioxaspiro[4.5]decane; as well as pharmaceutically acceptable salts, complexes, hydrates, solvates, or polymorphs, and pharmaceutically active derivative thereof.

12. A trioxolane according to any one of claims 1 to 11, wherein the pharmaceutically acceptable salts are selected from mesylate and tosylate.

13. A trioxolane according to any one of claims 1 to 11, wherein selected from the following group: *cis*-Adamantane-2-spiro-3'-8'-[4'-[(4'-hydroxy-2',2',6',6'-tetramethyl-4'-piperidinyl) methoxy]phenyl]-1',2',4'-trioxaspiro[4.5]decane *p*-tosylate and *cis-*Adamantane-2-spiro-3'-8'-[4'-[2'-(4'-piperidinyl)ethoxy]phenyl]-1',2',4'-trioxaspiro [4.5]decane mesylate; as well as pharmaceutically acceptable salts, complexes, hydrates, solvates, or polymorphs, and pharmaceutically active derivative thereof.

14. A trioxolane according to any one of claims 1 to 13 for use as a medicament.

15. A pharmaceutical formulation containing at least one trioxolane according to any one of claims 1 to 13 and a pharmaceutically acceptable carrier, diluent or excipient thereof
